# EUROPÄISCHE PATENTSCHRIFT

(11) **EP 2 012 092 B1**
(45) Veröffentlichungstag und Bekanntmachung des Hinweises auf die Patenterteilung: **01.12.2010**
(21) Anmeldenummer: 07013079.4
(22) Anmeldetag: 04.07.2007
(51) Int. Cl.: G01D 3/08, G01N 33/543

(54) **Messvorrichtung mit mindestens zwei Sensoren**
Measuring device with at least two sensors
Dispositif de mesure doté d'au moins deux capteurs

(43) Veröffentlichungstag der Anmeldung: 07.01.2009
(73) Patentinhaber: Micronas GmbH, 79108 Freiburg i. Br. (DE)
(72) Erfinder: Lehmann, Mirko, 79117 Freibung (DE); Freund, Ingo, 79117 Freiburg (DE); Mohry, Sonja, 79108 Freiburg (DE); Klapproth, Holger, 79108 Freiburg (DE)
(74) Vertreter: Koch Müller Patentanwaltsgesellschaft mbH

(56) Entgegenhaltungen:
- WO-A-02/33397
- WO-A-2004/025223
- WO-A-2006/005093
- DE-A1- 3 243 540
- DE-A1- 3 340 207
- DE-A1- 4 341 597
- DE-A1- 10 064 859

## Beschreibung

Die Erfindung betrifft eine Messvorrichtung mit mindestens einem Sensor zur Erfassung einer Messgröße, mit einem Datenspeicher und mit einer Stromversorgung dafür.

Eine derartige Messvorrichtung ist aus US 2006/0281435 A1 bekannt. Sie hat einen mit einem Datenspeicher verbundenen integrierten Schaltkreis, einen biologischen Sensor und eine Stromversorgung, die Mittel zur Erzeugung von elektrischer Energie aus einem in der Umgebung der Messvorrichtung vorhandenen elektromagnetischen Feld aufweist. Mit Hilfe der Stromversorgung werden der integrierte Schaltkreis und der Datenspeicher praktisch über die gesamte Lebensdauer der Messvorrichtung permanent mit elektrischer Energie versorgt. Somit kann die Messvorrichtung ohne externe Stromversorgungskabel betrieben werden. Die Messvorrichtung hat jedoch den Nachteil, dass die Messcharakteristik des biologischen Sensors einem Alterungsprozess und somit - über die Lebensdauer des Sensors betrachtet - entsprechend großen Toleranzen unterliegt. Das Messsignal des Sensors ist daher mit einem relativ großen Messfehler behaftet.

Aus DE 38 09 107 A1 ist ferner eine Messvorrichtung der eingangs genannten Art bekannt, die einen Gassensor hat, dessen Messcharakteristik von einer Reihe von Parametern, wie Temperatur, Feuchtigkeit und Druck beeinflusst wird und von Zeit zu Zeit nachgeeicht werden muss. Zu diesem Zweck wird der Sensor an Prüfzeitpunkten mittels eines in seinem Messkreis eingeprägten elektrischen Impulses zur Erzeugung eines eigenen Stromimpulses angeregt, dessen Form mit der Form des eingeprägten Stromimpulses verglichen wird. Aus diesem Vergleich werden die aktuellen Kennwerte des Sensors abgeleitet und in einem Datenspeicher abgelegt. Die Messvorrichtung hat den Nachteil, dass sie zu den Prüfzeitpunkten nicht für eine Messung der Gaskonzentration verwendet werden kann. Außerdem ist das aus DE 38 09 107 A1 bekannte Verfahren nur für Sensoren geeignet, bei denen sich unter dem Einfluss der physikalischen Größe der elektrische Innenwiderstand ändert.

Aus DE 43 41 597 A1 ist eine Messeinrichtung zur Kalibrierung und Erhöhung der Messgenauigkeit elektrochemischer Sensoren bekannt, bei der für eine Teilmenge von Sensoren aus einer Produktionsserie die herstellungsbedingte Exemplarstreuung sowie der Einfluss von Querempfindlichkeiten und Umgebungsparametern auf das Sensorsignal experimentell ermittelt und die daraus bestimmten Einflüsse in einem empirischen Korrekturansatz durch Anwendung eines genetischen Algorithmus berücksichtigt werden.

DE 100 64 859 A1 offenbart einen Sensor mit Hilfssensor zur Selbstkalibrierung.

Aus DE 33 40 207 A1 ist ein Verfahren zur Bestimmung einer Messgrösse mit einem ersten Sensor und der automatischen Erfassung der Temperaturahängigkeit von Messsignalen mit einem zweiten Sensor bekannt.

WO 2004/025223 A2 offenbart eine Vorrichtung zur Funktionsüberwachung von Sensoren, wobei eine zeitliche Entwicklung der gespeicherten Prüfparameter ausgewertet wird und daraus die zukünftige zu erwartende Entwicklung des Sensorverhaltens vorhergesagt wird und Informationen über die Dauer des verbleibenden störungsfreien Betriebs des Sensors gewonnen werden.

Es besteht deshalb die Aufgabe, eine Messvorrichtung der eingangs genannten Art zu schaffen, die es ermöglicht, eine Veränderung der Messcharakteristik des Sensors zu registrieren.

Diese Aufgabe wird durch die Messvorrichtung gemäss Anspruch 1 gelöst. Dabei wird unter einer dauerhaften Veränderung eine solche verstanden, bei der die Änderung der Messcharakteristik des ersten Sensors auch nach Entfernen der Einwirkung der physikalischen Größe auf den ersten Sensor zumindest teilweise und insbesondere irreversibel erhalten bleibt.

Der Erfindung liegt die Erkenntnis zugrunde, dass eine dauerhafte Veränderung der Messcharakteristik eines Sensors im Wesentlichen auf die Einwirkung einer oder mehrerer physikalischer Größen auf den Sensor zurückzuführen ist, und dass durch permanente Messung dieser Größe(n) mittels wenigstens eines zweiten Sensors und Auswertung dessen Messsignals (deren Messsignale) die Veränderung der Messcharakteristik bestimmt werden kann. Dabei kann die permanente Messung eine kontinuierliche Messung oder eine diskontinuierliche Messung sein. Bei letzterer ist das Zeitintervall zwischen zwei aufeinander folgenden Messzeitpunkten vorzugsweise so kurz gewählt ist, dass innerhalb des Zeitintervalls keine oder nur eine geringe Veränderung der physikalischen Messgröße(n) auftritt. Mit Hilfe der Auswerteeinrichtung wird die durch die physikalische(n) Größe(n) bewirkte Änderung der Messcharakteristik des ersten Sensors ermittelt und im Datenspeicher abgelegt. Dabei kann der Datenspeicher im Minimalfall eine Kapazität von nur einem Bit aufweisen, wenn in dem Datenspeicher eine Binärinformation abgelegt wird, die anzeigt, ob sich die Messcharakteristik des ersten Sensors innerhalb eines vorbestimmten Änderungsbereichs befindet oder nicht. Bevorzugt weist der Datenspeicher jedoch eine größere Speicherkapazität auf, damit beim Detektieren einer Änderung der Messcharakteristik des ersten Sensors diese mit entsprechend großer Genauigkeit in dem Datenspeicher abgelegt werden kann. Selbstverständlich ist es auch möglich, anstelle eines Kennwerts für die Änderung der Messcharakteristik direkt das Messsignal des zweiten Sensors im Datenspeicher zu archivieren und dann aus dem zeitlichen Verlauf des gespeicherten Messsignals mittels der Auswerteeinrichtung die Änderung der Messcharakteristik des ersten Sensors zu ermitteln.

Bei der Erfindung weist die Auswerteeinrichtung ein Integrationsglied zur Bildung eines Integrationssignals aus dem Messsignal des zweiten Sensors auf, wobei die Auswerteeinrichtung derart ausgebildet ist, dass sie die Änderung der Messcharakteristik des ersten Sensors in Abhängigkeit von dem Integrationssignal registriert. Mit Hilfe der Integrationseinrichtung können bei der Bestimmung der durch die Einwirkung der mindestens einen physikalischen Größe auf den ersten Sensor verursachten Veränderung dessen Messcharakteristik die Dauer, über welche die physikalischen Größe auf den ersten Sensor einwirkt, und die Amplitude des Messsignals der physikalischen Größe berücksichtigt werden.

Bei einer bevorzugten Ausgestaltung der Erfindung sind im Datenspeicher Kenngrößen abgelegt, die Wertekombinationen aufweisen, die jeweils zumindest einen Messsignal-Wert oder einem Messsignal-Wertebereich des zweiten Sensors und einen Wert für die Änderung der Messcharakteristik des ersten Sensors aufweisen, wobei die Auswerteeinrichtung zum Ermitteln der durch die Einwirkung der mit dem zweiten Sensor messbaren physikalischen Größe verursachten Änderung der Messcharakteristik des ersten Sensors mit dem Datenspeicher verbunden ist. Mit Hilfe der Kenngrößen kann die Änderung der Messcharakteristik des ersten Sensors mit großer Präzision bestimmt werden.

Vorteilhaft ist, wenn die Messvorrichtung einen Zeitgeber zum Ermitteln des Alters des ersten Sensors aufweist, wenn die Kenngrößen Wertekombinationen für mindestens zwei unterschiedliche Altersbereiche des ersten Sensors umfassen, und wenn die den Altersbereichen zugeordneten Wertekombinationen in Abhängigkeit von einem Zeitmesssignal des Zeitgebers aus dem Datenspeicher abrufbar sind. Bei der Auswertung des Messsignals des mindestens einen zweiten Sensors kann dann die vom Alter des ersten Sensors abhängige Empfindlichkeit des ersten Sensors gegenüber der mit dem zweiten Sensor messbaren physikalischen Größe berücksichtigt werden. Dabei ist es sogar möglich, dass die Einwirkung der physikalischen Messgröße je nach Alter des ersten Sensors dessen Messempfindlichkeit entweder reduziert oder erhöht.

Zweckmäßigerweise sind die Kenngrößen in Form mindestens einer Kennlinie und/oder mindestens eines Kennfelds in dem Datenspeicher abgelegt. Die mindestens eine Kennlinie kann eine Gerade sein und/oder einen progressiven und/oder degressiven Verlauf oder Abschnitt aufweisen. Die Kennlinie kann auch ein Polynom n-ten Gerades sein, wobei n eine ganze positive Zahl ist. Es ist auch möglich, dass die Kennlinie einen logarithmischen oder exponentiellen Verlauf aufweist. Das Kennfeld kann in Form von Stützstellen in dem Datenspeicher abgelegt sein. Zwischenstellen können bei Bedarf mit Hilfe der Auswerteeinrichtung aus den Stützstellen interpoliert werden.

Wenn der erste Sensor zur Erfassung der Messgröße biologisches Material aufweist, ist es vorteilhaft ist, wenn die Integrationseinrichtung eine nichtlineare, vorzugsweise progressive und insbesondere logarithmische Charakteristik aufweist. Dadurch kann die bei Einwirkung der physikalischen Größe auf das biologische Material etwa logarithmisch mit dem Intergral der physikalischen Größe abfallende Empfindlichkeit des biologischen Materials bei der Bestimmung der Änderung der Messcharakteristik des ersten Sensors berücksichtigt werden.

Bei einer vorteilhaften Ausgestaltung der Erfindung hat die Messvorrichtung mindestens ein Multiplikationsglied, das einen mit dem Messsignalausgang des zweiten Sensors oder einem Anschluss für das Integrationssignal verbundenen ersten Multiplikationseingang und einen mit dem Datenspeicher verbundenen zweiten Multiplikationseingang aufweist. Das Messsignal des zweiten Sensors kann dann mit in dem Datenspeicher abgelegten, von der Amplitude des Messsignals abhängigen Faktoren gewichtet werden, bevor es aufintegriert wird. Die Auswahl der Faktoren kann auch in Abhängigkeit von mindestens einem weiteren Messsignal erfolgen, um beispielsweise eine dauerhafte oder irreversible Änderung der Messcharakteristik, die durch gleichzeitiges Einwirken mehrer unterschiedlicher physikalischer Größen auf den ersten Sensor verursacht ist, genauer bestimmen zu können. Das Multiplikationsglied kann auch in einen Mikrocomputer integriert sein, auf dem ein entsprechendes Betriebsprogramm ablaufbar ist.

Besonders vorteilhaft ist, wenn die Messvorrichtung wenigstens eine Justiereinrichtung für den mindestens ersten Sensor aufweist, mittels der die Messcharakteristik des ersten Sensors verstellbar ist, und wenn die Auswerteeinrichtung derart mit der Justiereinrichtung in Steuerverbindung steht, dass beim Registrieren einer Änderung der Messcharakteristik des ersten Sensors diese mittels der Justiereinrichtung Im Sinne einer Reduzierung dieser Änderung verstellt wird. Somit kann die durch die Einwirkung der physikalischen Größe(n) bewirkte dauerhafte Änderung der Messcharakteristik des ersten Sensors zumindest teilweise wieder kompensiert werden.

Bei einer bevorzugten Ausführungsform der Erfindung ist der mindestens eine erste Sensor ein Biochip, der einen Träger aufweist, auf dem an mindestens einer Teststelle wenigstens ein Rezeptor immobilisiert ist, der für einen nachzuweisenden Liganden bindungsspezifisch ist. Dabei kann der Rezeptor eine Nukleinsäure oder ein Derivat davon (DNA, RNA, PNA, LNA, Oligonukleotide, Plasmide, Chromosomen), ein Peptid, Protein (Enzym, Protein, Oligopeptide, zelluläre Rezeptorproteine und deren Komplexe, Peptidhormone, Antikörper und deren Fragmente), Kohlenhydrat und dessen Derivate, insbesondere ein glykosyliertes Protein und Glycosid, Fett, Fettsäure und/oder Lipid umfassen.

Bei einer solchen Messvorrichtung umfasst der mindestens eine zweite Sensor zweckmäßigerweise zumindest einen Temperatur-, Feuchtigkeits- und/oder Strahlungssensor. Dadurch können die für die Veränderung der Messcharakteristik des Biochips wesentlichen Einflussgrößen, nämlich die Temperatur, die Feuchtigkeit und das Licht bzw. die Helligkeit in der Umgebung der Rezeptoren, bei der Ermittlung der Änderung der Messcharakteristik und ggf deren Kompensation berücksichtigt werden.

Vorteilhaft ist, wenn der wenigstens eine Rezeptor in stabilisierter Form auf dem Träger immobilisiert ist. Durch die Stabilisierung wird die Haltbarkeit der Rezeptoren verbessert. Außerdem wird der Temperaturbereich, dem die Rezeptoren ausgesetzt sein können, ohne dass sich die Messcharakteristik des den Rezeptor aufweisenden ersten Sensors wesentlich verändert, erweitert. Daher ist eine Überwachung der mindestens einen physikalischen Größe, die dazu geeignet ist, die Messcharakteristik des ersten Sensors dauerhaft zu verändern, in diesem Fall von besonderer Bedeutung. Die Stabilisierung der Rezeptoren kann insbesondere dadurch erreicht werden, dass der auf dem Träger immobilisierte Rezeptor bei der Herstellung des Biochips mit einer Zusammensetzung beaufschlagt wird, die mindestens ein nichtreduziertes Disaccarid und mindestens ein Protein oder Polypeptid der LEA-Klasse umfasst. Entsprechende Disaccaride und Proteine sind aus EP 1 534 740 B1 bekannt.

Zweckmäßigerweise weist der Biochip mindestens zwei vorzugsweise seitlich voneinander beabstandete Teststellen auf, an denen jeweils ein erster Sensor angeordnet ist, wobei in dem Datenspeicher für jede dieser Teststellen jeweils mindestens eine Kenngröße abgelegt ist. Dadurch kann insbesondere berücksichtigt werden, dass sich die Rezeptoren bei Wärme- und/oder Kälteeinwirkung unterschiedlich verändern. So kann es beispielsweise sein, dass ein an einer ersten Teststelle angeordneter erster Rezeptor nach einer Temperatureinwirkung ein größeres und ein an einer zweiten Teststelle angeordneter zweiter Rezeptor ein kleineres Messsignal zeigt also vor der Temperatureinwirkung. Auch kann durch die für die einzelnen Teststellen getrennt in dem Datenspeicher abgelegten Kenngrößen berücksichtigt werden, dass unterschiedliche, an den einzelnen Teststellen als Rezeptoren dienende Antikörper Zerfallsreihen aufweisen können, die sich voneinander unterscheiden.

Bei einer Vorteilhaften Ausgestaltung der Erfindung ist der Träger ein Halbleiterchip, in den mindestens ein Sensorelement integriert ist, dessen Messsignal von der Bindung des Liganden an den Rezeptor abhängig ist. Dabei kann das Sensorelement ein optischer Sensor zur Detektion einer in Abhängigkeit von der Bindung des Liganden an den Rezeptor erzeugten Lumineszenzstrahlung und/oder ein ionensensitiver Feldeffettransistor (ISFET) sein.

Die zumindest für den zweiten Sensor und den Datenspeicher vorgesehene Stromversorgung kann eine elektrische Energiequelle, insbesondere eine Batterie und/oder eine Brennstoffzelle aufweisen. Dabei ist es sogar möglich, dass die Brennstoffzelle in den Halbleiterchip integriert ist, so dass sichergestellt ist, dass der erste Sensor bereits ab seiner Fertigung bezüglich der Einwirkung der physikalischen Größe(n) überwacht wird. Außerdem ermöglicht die in den Halbleiterchip integrierte Brennstoffzelle einen einfachen, kostengünstigen und kompakten Aufbau der Messvorrichtung. Die Brennstoffzelle kann insbesondere die aus DE 102 55 736 A1 bekannten Merkmale aufweisen.

Bei einer anderen vorteilhaften Ausgestaltung der Erfindung weist die Stromversorgung eine Einrichtung zur Erzeugung von elektrischer Energie aus optischer Strahlung, aus elektromagnetischer Strahlung und/oder aus mechanischer Bewegungsenergie auf Diese Einrichtung ist vorzugsweise mit Methoden der Halbleiterfertigung und/oder Mikrosystemtechnik in den Halbleiterchip integriert. Sie kann insbesondere eine Photozelle, Mittel zum Umwandeln von Radiowellen in eine elektrische Gleichspannung und/oder einen piezoelektrischen Wandler umfassen.

Nachfolgend sind Ausführungsbeispiele der Erfindung anhand der Zeichnung näher erläutert. Es zeigt:
- Fig. 1: ein Blockschaltbild eines ersten Ausführungsbeispiels einer Messvorrichtung, die mehrere matrixförmig angeordnete erste Sensoren und zweite Sensoren aufweist,
- Fig.2: einen Teilquerschnitt durch die Messvorrichtung, wobei zwei erste Sensoren erkennbar sind, an denen in Abhängigkeit von der Bindung eines Liganden an einen Rezeptor eine Lumineszenzstrahlung erzeugt wird,
- Fig. 3: einen Teilquerschnitt durch eine Messvorrichtung, bei der die Lumineszenzstrahlung chemisch erzeugt wird,
- Fig. 4: ein Blockschaltbild eines zweiten Ausführungsbeispiels der Messvorrichtung,
- Fig. 5: eine graphische Darstellung des Leuchtdichtesignals einer optischen Strahlung, die in Abhängigkeit von der Bindung eines Liganden an einen auf einem Träger immobilisierten Rezeptor eines Biochips erzeugt wird, wobei auf der Abzisse die Lagerdauer t des Biochips bei einer vorbestimmten, konstanten Temperatur und auf der Ordinate das Leuchtdichtesignal S aufgetragen ist, wobei die quadratisch umrahmten Stellen Messpunkte eines Biochips mit nicht stabilisiertem Rezeptor und die Vollquadrate Messpunkte eines Biochips mit stabilisiertem Rezeptor markieren.

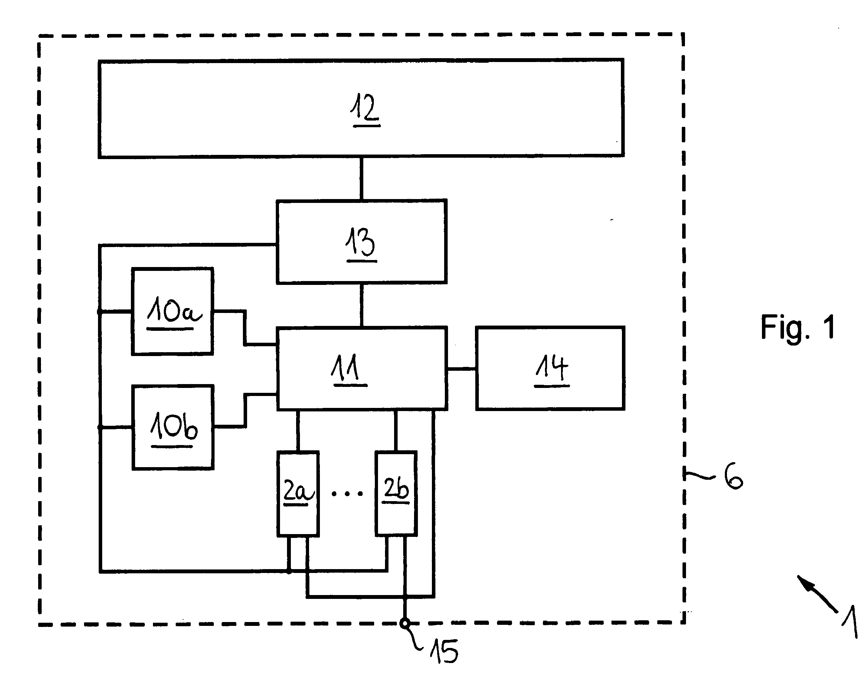

Eine in Fig. 1 im Ganzen mit 1 bezeichnete Messvorrichtung hat mehrere erste Sensoren 2a, 2b, die jeweils ein optisches Sensorelement 3 und mindestens einen diesem zugeordneten Rezeptor 4 aufweisen, der für einen vorbestimmten, in einem Analyt nachzuweisenden und/oder bezüglich seiner Konzentration zu bestimmenden Ligand 5 bindungsspezifisch ist.

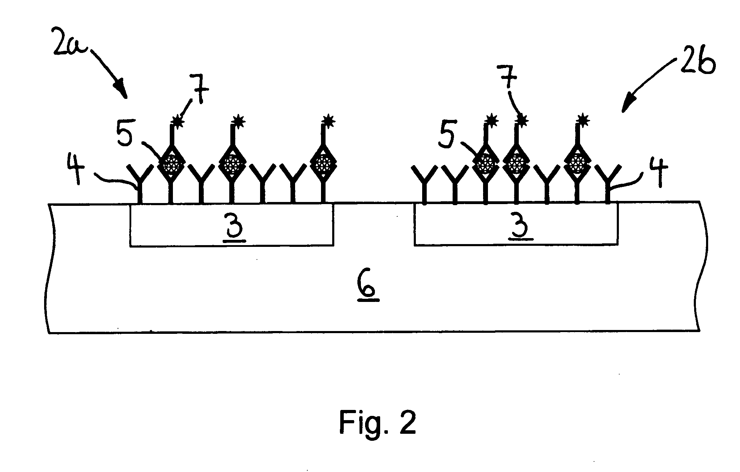

Wie in Fig. 2 erkennbar ist, sind die Rezeptoren 4 an Teststellen immobilisiert, die auf einem flächigen Träger 6 angeordnet sind. Die Rezeptoren 4 von mindestens zwei Teststellen sind für unterschiedliche Liganden 5 bindungsspezifisch. Die einzelnen Teststellen sind seitlich durch rezeptorfreie Bereiche voneinander beabstandet.

Zur Untersuchung des Analyts wird dieser derart mit den Teststellen in Kontakt gebracht, dass die in dem Analyten enthaltenen Liganden 5 die Möglichkeit haben, spezifisch an die Rezeptoren 4 zu binden. Die Liganden 5 sind mit einem Marker 7 markiert, der durch Bestrahlung mit einer Anregungsstrahlung zur Aussendung von Lumineszenzstrahlung 8 angeregt wird. Die Wellenlänge der Lumineszenzstrahlung 8 unterscheidet sich von derjenigen der Anregungsstrahlung.

In Fig. 2 ist erkennbar, dass das Sensorelement 3 jeweils direkt unter dem mindestens einen ihm zugeordneten Rezeptor 4 angeordnet und in den zumindest bereichsweise als Halbleiterchip ausgebildeten Träger 6 integriert ist. Die Sensorelemente 3 sind für die von den Markern 7 ausgesandte Lumineszenzstrahlung 8 empfindlich und für die Anregungsstrahlung unempfindlich.

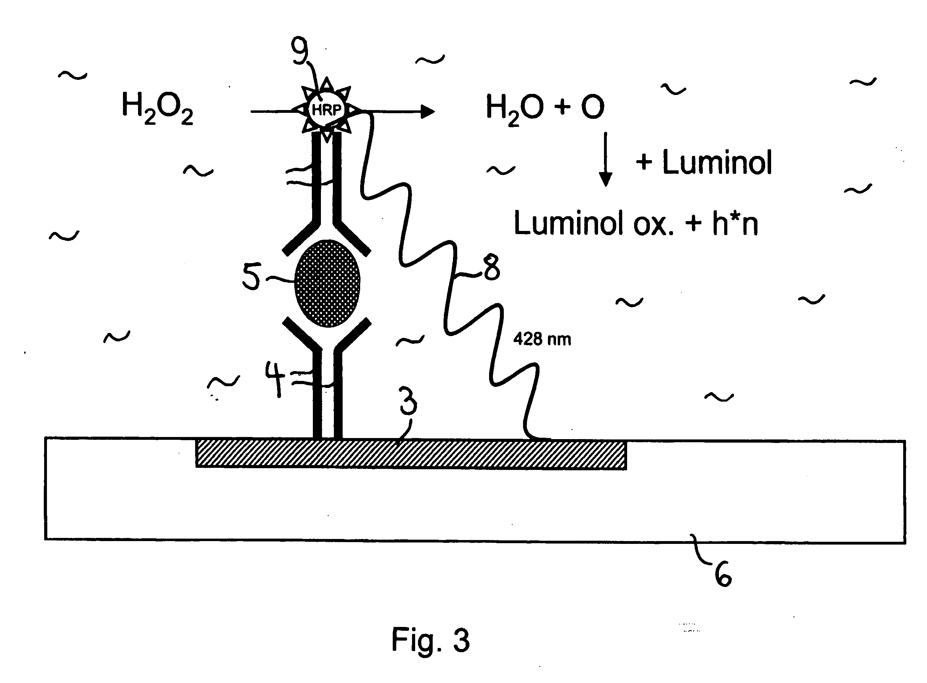

Bei dem in Fig. 3 dargestellten Ausführungsbeispiel wird die Lumineszenzstrahlung 8 chemisch in Abhängigkeit von der Bindung des Liganden 5 an den Rezeptor 4 erzeugt. Dabei ist der Ligand 5 mit einem Enzym 9 markiert. Dem Analyt sind Luminol und Wasserstoffperoxid beigemischt. Bei Anwesenheit des Enzyms 9 tritt zwischen dem Luminol und dem Wasserstoffperoxid eine chemische Reaktion auf, bei der das Luminol unter Abgabe von Lumineszenzstrahlung 8 oxidert wird. Wie bei dem in Fig. 2 gezeigten Ausführungsbeispiel ist das für die Lumineszenzstrahlung 8 empfindliche Sensorelement 3 auch in Fig. 3 direkt unter dem mindestens einen Rezeptor 4 angeordnet.

Die Messvorrichtung weist zusätzlich zu den ersten Sensoren 2a, 2b zweite Sensoren 10a, 10b auf, die zur Erfassung von auf die ersten Sensoren 2a, 2b einwirkenden physikalischen Größen vorgesehen sind, die dazu geeignet sind, die Messcharakteristik des ersten Sensors 2a, 2b dauerhaft zu verändern. Ein erster zweiter Sensor 10a ist als Temperatursensor und ein weiterer zweiter Sensor 10b als Feuchtigkeitssensor ausgestaltet. Die zweiten Sensoren 10a, 10b sind ebenfalls in den Träger 6 integriert.

Zur Bestimmung einer durch die Einwirkung der genannten physikalischen Größen verursachten dauerhaften Änderung der Messcharakteristik mindestens eines ersten Sensors 2a, 2b ist eine Auswerteeinrichtung 11 vorgesehen, die mit Messsignalausgängen der zweiten Sensoren 10a, 10b verbundene Eingänge aufweist. Die Auswerteeinrichtung 11 hat einen in der Zeichnung nicht näher dargestellten Mikroprozessor, der durch ein in einem Programmspeicher abgelegtes Betriebsprogramm gesteuert wird. Zur Messung des Alters der ersten Sensoren 2a, 2b ist in den Träger 6 ein Zeitgeber 13 integriert.

Zusätzlich zu dem Programmspeicher ist ein Datenspeicher 14 vorgesehen, der über einen elektronischen Bus mit der Auswerteeinrichtung 11 verbunden ist. Über die Auswerteeinrichtung 11 ist der Datenspeicher 14 außerdem mit den zweiten Sensoren 10a, 10b verbunden. In dem Datenspeicher sind für jeden ersten Sensor 2a, 2b jeweils Kenngrößen abgelegt, die Wertekombinationen aufweisen, die Messsignal-Werten oder Messsignal-Wertebereichen der zweiten Sensoren 10a, 10b für unterschiedliche Altersbereiche der ersten Sensoren 2a, 2b jeweils einen Wert für die Änderung der Messcharakteristik des betreffenden ersten Sensors 2a, 2b zuordnen.

Zur Versorgung der ersten Sensoren 2a, 2b, der zweiten Sensoren 10a, 10b, der Auswerteeinrichtung 11 und des Datenspeichers 14 mit elektrischer Energie ist in den Träger eine Stromversorgung 12 integriert, die als Brennstoffzelle ausgestaltet und über in der Zeichnung nicht näher dargestellte elektrische Leitungen mit den Sensoren 2a, 2b, 10a, 10b, der Auswerteeinrichtung 11, dem Zeitgeber 13 und dem Datenspeicher 14 verbunden ist.

An der Stromversorgung 12 ist außerdem der Zeitgeber 13 angeschlossen, der in vorbestimmten Zeitabständen Messzyklen startet, bei denen mit Hilfe jedes zweiten Sensors 10a, 10b jeweils mindestens ein Messwert erfasst und an die Auswerteeinrichtung 11 weitergeleitet wird. Für jeden ersten Sensor 2a, 2b wird jeweils der den jeweiligen Messwerten zugeordnete Wert für die Änderung der Messcharakteristik des betreffenden ersten Sensors 2a, 2b aus dem Datenspeicher 14 ausgelesen. Die so für jeden ersten Sensor 2a, 2b jeweils erhaltenen Werte werden in der Auswerteeinrichtung 11 aufintegriert, um einen Statuswert zu bestimmen. Die Statuswerte werden in dem Datenspeicher 14 abgelegt. Sie können über einen mit dem Datenspeicher 14 verbundenen elektrischen Anschluss 15 aus der Messvorrichtung 1 ausgelesen werden, um die jeweils aktuelle Messcharakteristik der ersten Sensoren 2a, 2b abzufragen und/oder zu überprüfen, ob die einzelnen ersten Sensoren 2a, 2b noch für eine Messung brauchbar sind. Zum Auslesen der Messsignale aus den ersten Sensoren 2a, 2b sind diese ebenfalls mit dem Anschluss 15 verbunden.

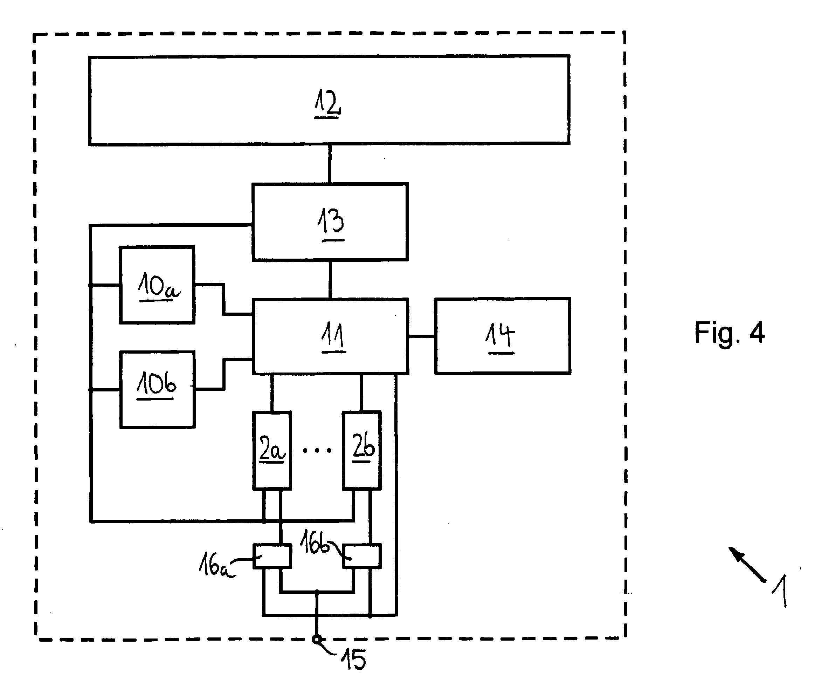

Bei dem in Fig. 4 gezeigten Ausführungsbeispiel weist die Messvorrichtung 1 für jeden ersten Sensor 2a, 2b jeweils eine Justiereinrichtung 16a, 16b auf, mittels der die Messcharakteristik des betreffenden ersten Sensors 2a, 2b verstellbar ist. Die Justiereinrichtung 16a, 16b kann beispielsweise einen zwischen dem Messsignalausgang des betreffenden ersten Sensors 2a, 2b und dem Anschluss 15 angeordneten steuerbareren Verstärker und/oder Abschwächer aufweisen. Die Auswerteeinrichtung 11 steht derart mit den Justiereinrichtungen 16a, 16b in Steuerverbindung, dass beim Registrieren einer Änderung der Messcharakteristik eines ersten Sensors 2a, 2b die diesem Sensor 2a, 2b zugeordnete Justiereinrichtung im Sinne einer Reduzierung der Änderung verstellt wird.

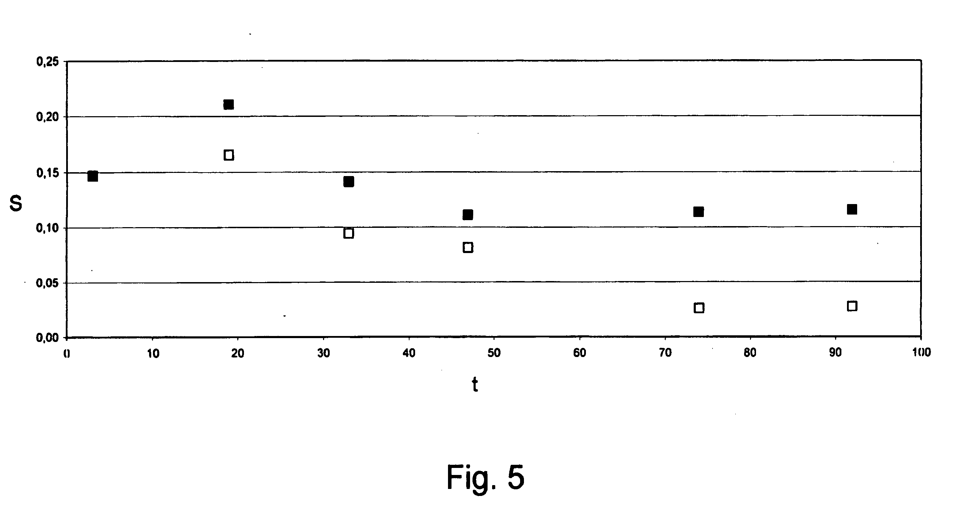

Die Rezeptoren 4 sind bevorzugt in stabilisierter Form auf dem Träger 6 immobilisiert. Dadurch wird gegenüber einer Messvorrichtung 1, bei der die Rezeptoren 4 nicht stabilisierter sind, insbesondere nach einer längeren Lagerdauer der Messvorrichtung 1 eine größere Messempfindlichkeit der ersten Sensoren 2a, 2b erreicht (Fig. 5).

## Patentansprüche

1. Messvorrichtung (1) mit mindestens einem Sensor (2a, 2b) zur Erfassung einer Messgröße, mit einem Datenspeicher (14) und mit einer Stromversorgung (12) dafür, wobei die Messvorrichtung (1) zusätzlich zu dem ersten Sensor (2a, 2b) mindestens einen mit der Stromversorgung (12) und dem Datenspeicher (14) verbundenen zweiten Sensor (1 0a, 10b) aufweist, der zur Erfassung einer auf den ersten Sensor (2a, 2b) einwirkenden physikalischen Größe vorgesehen ist, die dazu geeignet ist, die Messcharakterisfik des ersten Sensors (2a, 2b) dauerhaft zu verändern, und wobei mit dem zweiten Sensor (10a, 10b) und dem Datenspeicher (14) eine Auswerteeinrichtung (11) derart zusammenwirkt, dass in Abhängigkeit vom Messsignal des zweiten Sensors (1 0a, 10b) die Änderung der Messcharakteristik des ersten Sensors (2a, 2b) registrierbar ist **dadurch gekennzeichnet, dass** die Auswerteeinrichtung (11) eine Integrationseinrichtung zur Bildung eines Integrafionssignals aus dem Messsignal des zweiten Sensors (1 0a, 10b) aufweist, und dass die Auswerteeinrichtung (11) derart ausgebildet ist, dass sie die Änderung der Messcharakteristik des ersten Sensors (2a, 2b) in Abhängigkeit von dem Integrafionssignal registriert.

2. Messvorrichtung (1) nach Anspruch 1, **dadurch gekennzeichnet, dass** im Datenspeicher (14) Kenngrößen abgelegt sind, die Wertekombinationen aufweisen, die jeweils zumindest einen Messsignal-Wert oder einem Messsignal-Wertebereich des zweiten Sensors (1 0a, 10b) und einen Wert für die Änderung der Messcharakteristik des ersten Sensors (2a, 2b) aufweisen, und dass die Auswerteeinrichtung (11) zum Ermitteln der durch die Einwirkung der mit dem zweiten Sensor (10a, 10b) messbaren physikalischen Größe verursachten Änderung der Messcharakteristik des ersten Sensors (2a, 2b) mit dem Datenspeicher (14) verbunden ist.

3. Messvorrichtung (1) nach Anspruch 1 oder 2, **dadurch gekennzeichnet, dass** sie einen Zeitgeber (1 3) zum Ermitteln des Alters des ersten Sensors (2a, 2b) aufweist, dass die Kenngrößen Wertekombinationen für mindestens zwei unterschiedliche Altersbereiche des ersten Sensors (2a, 2b) umfassen, und dass die den Altersbereichen zugeordneten Wertekombinationen in Abhängigkeit von einem Zeitmesssignal des Zeitgebers (1 3) aus dem Datenspeicher (14) abrufbar sind.

4. Messvorrichtung (1) nach einem der Ansprüche 2 oder 3, **dadurch gekennzeichnet, dass** die Kenngrößen in Form mindestens einer Kennlinie und/oder mindestens eines Kennfelds in dem Datenspeicher (14) abgelegt sind.

5. Messvorrichtung (1) nach einem der Ansprüche 1 bis 4, **dadurch gekennzeichnet, dass** die Integrationseinrichtung eine nichtlineare, vorzugsweise progressive und insbesondere logarithmische Charakteristik aufweist.

6. Messvorrichtung (1) nach einem der Ansprüche 1 bis 5, **dadurch gekennzeichnet, dass** die Messvorrichtung (1) mindestens ein Multiplikationsglied aufweist, das einen mit dem Messsignalausgang des zweiten Sensors (10a, 10b) oder einem Anschluss für das Integrationssignal verbundenen ersten Mulfiplikafionseingang und einen mit dem Datenspeicher (14) verbundenen zweiten Mulfiplikafionseingang aufweist.

7. Messvorrichtung (1) nach einem der Ansprüche 1 bis 6, **dadurch gekennzeichnet, dass** die Messvorrichtung (1) wenigstens eine Justiereinrichtung (16a, 16b) für den mindestens ersten Sensor (2a, 2b) aufweist, mittels der die Messcharakteristik des ersten Sensors (2a, 2b) verstellbar ist, und dass die Auswerteeinrichtung (11) derart mit der Justiereinrichtung (16a, 16b) in Steuerverbindung steht, dass beim Registrieren einer Änderung der Messcharakteristik des ersten Sensors (2a, 2b) diese mittels der Justiereinrichtung (16a, 16b) im Sinne einer Reduzierung dieser Änderung verstellt wird.

8. Messvorrichtung (1) nach einem der Ansprüche 1 bis 7, **dadurch gekennzeichnet, dass** der mindestens eine zweite Sensor (1 0a, 10b) zumindest einen Temperatur-, Feuchfigkeits- und/oder Strahlungssensor umfasst.

9. Messvorrichtung (1) nach einem der Ansprüche 1 bis 8, **dadurch gekennzeichnet, dass** der mindestens eine erste Sensor (2a, 2b) ein Biochip ist, der einen Träger (6) aufweist, auf dem an mindestens einer Teststelle wenigstens ein Rezeptor (4) immobilisiert ist, der für einen nachzuweisenden Liganden (5a, 5b) bindungsspezifisch ist.

10. Messvorrichtung (1) nach Anspruch 9, **dadurch gekennzeichnet, dass** der wenigstens eine Rezeptor (4) in stabilisierter Form auf dem Träger (6) immobilisiert ist.

11. Messvorrichtung (1) nach Anspruch 9 oder 10, **dadurch gekennzeichnet, dass** der Biochip mindestens zwei vorzugsweise seitlich voneinander beabstandete Teststellen aufweist, an denen jeweils ein erster Sensor (2a, 2b) angeordnet ist, und dass in dem Datenspeicher (14) für jede dieser Teststellen jeweils mindestens eine Kenngröße abgelegt ist.

12. Messvorrichtung (1) nach einem der Ansprüche 9 bis 11, **dadurch gekennzeichnet, dass** der Träger (6) ein Halbleiterchip ist, in den mindestens ein Sensorelement (3) integriert ist, dessen Messsignal von der Bindung des Liganden (5) an den Rezeptor (4) abhängig ist.

13. Messvorrichtung (1) nach einem der Ansprüche 1 bis 12, **dadurch gekennzeichnet, dass** die Stromversorgung (12) eine elektrische Energiequelle, insbesondere eine Batterie und/oder eine Brennstoffzelle aufweist.

14. Messvorrichtung (1) nach einem der Ansprüche 1 bis 13, **dadurch gekennzeichnet, dass** die Stromversorgung eine Einrichtung zur Erzeugung von elektrischer Energie aus optischer Strahlung, aus elektromagnefischer Strahlung und/oder aus mechanischer Bewegungsenergie aufweist.

## Claims

1. Measuring device (1) with at least one sensor (2a, 2b) for recording a measurement variable, with a data memory (14) and with a power supply (12) for the latter, the measuring device (1) having, in addition to the first sensor (2a, 2b), at least one second sensor (10a, 10b) which is connected to the power supply (12) and to the data memory (14) and is intended to record a physical variable which acts on the first sensor (2a, 2b) and is suitable for permanently changing the measurement characteristic of the first sensor (2a, 2b), and an evaluation device (11) interacting with the second sensor (10a, 10b) and the data memory (14) in such a manner that the change in the measurement characteristic of the first sensor (2a, 2b) can be recorded on the basis of the measurement signal from the second sensor (10a, 10b), **characterized in that** the evaluation device (11) has an integration device for forming an integration signal from the measurement signal from the second sensor (10a, 10b), and **in that** the evaluation device (11) is designed in such a manner that it records the change in the measurement characteristic of the first sensor (2a, 2b) on the basis of the integration signal.

2. Measuring device (1) according to Claim 1, **characterized in that** the data memory (14) stores characteristic variables which have combinations of values each having at least one measurement signal value or a range of measurement signal values of the second sensor (10a, 10b) and a value for the change in the measurement characteristic of the first sensor (2a, 2b), and **in that** the evaluation device (11) is connected to the data memory (14) in order to determine the change in the measurement characteristic of the first sensor (2a, 2b), which change is caused by the action of the physical variable which can be measured using the second sensor (10a, 10b).

3. Measuring device (1) according to Claim 1 or 2, **characterized in that** it has a timer (13) for determining the age of the first sensor (2a, 2b), **in that** the characteristic variables comprise combinations of values for at least two different age ranges of the first sensor (2a, 2b), and **in that** the combinations of values assigned to the age ranges can be retrieved from the data memory (14) on the basis of a time measurement signal from the timer (13).

4. Measuring device (1) according to either of Claims 2 and 3, **characterized in that** the characteristic variables are stored in the data memory (14) in the form of at least one characteristic curve and/or at least one family of characteristic curves.

5. Measuring device (1) according to one of Claims 1 to 4, **characterized in that** the integration device has a non-linear, preferably progressive and, in particular, logarithmic characteristic.

6. Measuring device (1) according to one of Claims 1 to 5, **characterized in that** the measuring device (1) has at least one multiplication element which has a first multiplication input, which is connected to the measurement signal output of the second sensor (10a, 10b) or to a connection for the integration signal, and a second multiplication input which is connected to the data memory (14).

7. Measuring device (1) according to one of Claims 1 to 6, **characterized in that** the measuring device (1) has at least one adjustment device (16a, 16b) for the at least first sensor (2a, 2b), which adjustment device can be used to adjust the measurement characteristic of the first sensor (2a, 2b), and **in that** the evaluation device (11) has a control connection to the adjustment device (16a, 16b) in such a manner that, when recording a change in the measurement characteristic of the first sensor (2a, 2b), said characteristic is adjusted using the adjustment device (16a, 16b) in the sense of a reduction in this change.

8. Measuring device (1) according to one of Claims 1 to 7, **characterized in that** the at least one second sensor (10a, 10b) comprises at least one temperature, humidity and/or radiation sensor.

9. Measuring device (1) according to one of Claims 1 to 8, **characterized in that** the at least one first sensor (2a, 2b) is a biochip having a carrier (6) on which at least one receptor (4) which binds specifically to a ligand (5a, 5b) to be detected is immobilized at at least one test site.

10. Measuring device (1) according to Claim 9, **characterized in that** the at least one receptor (4) is immobilized on the carrier (6) in stabilized form.

11. Measuring device (1) according to Claim 9 or 10, **characterized in that** the biochip has at least two test sites which are preferably laterally spaced apart from one another and at which a first sensor (2a, 2b) is respectively arranged, and **in that** the data memory (14) stores at least one respective characteristic variable for each of these test sites.

12. Measuring device (1) according to one of Claims 9 to 11, **characterized in that** the carrier (6) is a semiconductor chip in which at least one sensor element (3) is integrated, the measurement signal of said sensor element depending on the binding of the ligand (5) to the receptor (4).

13. Measuring device (1) according to one of Claims 1 to 12, **characterized in that** the power supply (12) has an electrical energy source, in particular a battery and/or a fuel cell.

14. Measuring device (1) according to one of Claims 1 to 13, **characterized in that** the power supply has a device for generating electrical energy from optical radiation, from electromagnetic radiation and/or from mechanical kinetic energy.

## Revendications

1. Dispositif de mesure (1) comprenant au moins un capteur (2a, 2b) pour acquérir une grandeur mesurée, comprenant une mémoire de données (14) et comprenant une alimentation électrique (12) à cet effet, le dispositif de mesure (1) présentant en plus du premier capteur (2a, 2b) au moins un deuxième capteur (10a, 10b) relié avec l'alimentation électrique (12) et la mémoire de données (14), lequel est prévu pour détecter une grandeur physique qui agit sur le premier capteur (2a, 2b) et qui est conçue pour modifier durablement la caractéristique de mesure du premier capteur (2a, 2b), et un dispositif d'interprétation (11) interagissant avec le deuxième capteur (10a, 10b) et la mémoire de données (14) de telle sorte que la modification de la caractéristique du premier capteur (2a, 2b) peut être enregistrée en fonction du signal de mesure du deuxième capteur (10a, 10b), **caractérisé en ce que** le dispositif d'interprétation (11) présente un dispositif d'intégration pour former un signal d'intégration à partir du signal de mesure du deuxième capteur (10a, 10b) et que le dispositif d'interprétation (11) est configuré de telle sorte qu'il enregistre la modification de la caractéristique de mesure du premier capteur (2a, 2b) en fonction du signal d'intégration.

2. Dispositif de mesure (1) selon la revendication 1, **caractérisé en ce que** des grandeurs caractéristiques sont stockées dans la mémoire de données (14), lesquelles présentent des combinaisons de valeurs qui présentent respectivement au moins une valeur du signal de mesure ou une plage de valeurs du signal de mesure du deuxième capteur (10a, 10b) et une valeur pour la modification de la caractéristique de mesure du premier capteur (2a, 2b), et que le dispositif d'interprétation (11) est relié avec la mémoire de données (14) afin de déterminer la modification de la caractéristique de mesure du premier capteur (2a, 2b) provoquée par l'effet de la grandeur physique mesurable avec le deuxième capteur (10a, 10b).

3. Dispositif de mesure (1) selon la revendication 1 ou 2, **caractérisé en ce qu'**il présente un générateur d'horloge (13) pour déterminer l'âge du premier capteur (2a, 2b), que les grandeurs caractéristiques incluent des combinaisons de valeurs pour au moins deux plages d'âge différentes du premier capteur (2a, 2b) et que les combinaisons de valeurs associées aux plages d'âges peuvent être invoquées depuis la mémoire de données (14) en fonction d'un signal de mesure du temps du générateur d'horloge (13).

4. Dispositif de mesure (1) selon l'une des revendications 2 ou 3, **caractérisé en ce que** les grandeurs caractéristiques sont stockées dans la mémoire de données (14) sous la forme d'au moins une courbe caractéristique et/ou d'au moins un champ caractéristique.

5. Dispositif de mesure (1) selon l'une des revendications 1 à 4, **caractérisé en ce que** le dispositif d'intégration présente une caractéristique non linéaire, de préférence progressive et notamment logarithmique.

6. Dispositif de mesure (1) selon l'une des revendications 1 à 5, **caractérisé en ce que** le dispositif de mesure (1) présente au moins un élément multiplicateur qui présente une première entrée de multiplication reliée avec la sortie signal de mesure du deuxième capteur (10a, 10b) ou une borne pour le signal d'intégration et une deuxième entrée de multiplication reliée avec la mémoire de données (14).

7. Dispositif de mesure (1) selon l'une des revendications 1 à 6, **caractérisé en ce que** le dispositif de mesure (1) présente au moins un dispositif de réglage (16a, 16b) pour l'au moins un premier capteur (2a, 2b), au moyen duquel la caractéristique de mesure du premier capteur (2a, 2b) peut être ajustée, et que le dispositif d'interprétation (11) se trouve en liaison de commande avec le dispositif de réglage (16a, 16b) de telle sorte que lors de l'enregistrement d'une modification de la caractéristique de mesure du premier capteur (2a, 2b), celle-ci est ajustée au moyen du dispositif de réglage (16a, 16b) dans le sens d'une réduction de cette modification.

8. Dispositif de mesure (1) selon l'une des revendications 1 à 7, **caractérisé en ce que** l'au moins un deuxième capteur (10a, 10b) comprend au moins un capteur de température, d'humidité et/ou de rayonnement.

9. Dispositif de mesure (1) selon l'une des revendications 1 à 8, **caractérisé en ce que** l'au moins un premier capteur (2a, 2b) est une biopuce qui présente un support (6) sur lequel au moins un récepteur (4) est immobilisé en au moins un point de test, ledit récepteur (4) étant spécifique à une liaison pour un ligand (5a, 5b) dont il faut détecter la présence.

10. Dispositif de mesure (1) selon la revendication 9, **caractérisé en ce que** l'au moins un récepteur (4) est immobilisé sur le support (6) sous une forme stabilisée.

11. Dispositif de mesure (1) selon la revendication 9 ou 10, **caractérisé en ce que** la biopuce présente au moins deux points de test de préférence espacés l'un de l'autre latéralement et auxquels est respectivement disposé un premier capteur (2a, 2b) et qu'au moins une grandeur caractéristique est à chaque fois stockée dans la mémoire de données (14) pour chacun de ces points de test.

12. Dispositif de mesure (1) selon l'une des revendications 9 à 11, **caractérisé en ce que** le support (6) est une puce en semiconducteur dans laquelle est intégré au moins un élément de détection (3) dont le signal de mesure est dépendant de la liaison du ligand (5) au niveau du récepteur (4).

13. Dispositif de mesure (1) selon l'une des revendications 1 à 12, **caractérisé en ce que** l'alimentation électrique (12) présente une source d'énergie électrique, notamment une batterie et/ou une pile à combustible.

14. Dispositif de mesure (1) selon l'une des revendications 1 à 13, **caractérisé en ce que** l'alimentation électrique présente un dispositif pour générer de l'énergie électrique à partir d'un rayonnement optique, d'un rayonnement électromagnétique et/ou de l'énergie cinétique mécanique.
